Europäisches Patentamt

European Patent Office

Office européen des brevets

(11) Publication number: **0 105 014 B1**

# EUROPEAN PATENT SPECIFICATION

(45) Date of publication of patent specification: **20.05.92**

(51) Int. Cl.⁵: **A61K 37/02**, A61K 35/12, A61K 47/00, C07K 7/00, C12N 15/00, C12P 21/02, C07C 233/00, C07C 237/00, A61K 37/26, //(A61K37/26, 37:02),(A61K37/02,31:56)

(21) Application number: **83450022.5**

(22) Date of filing: **22.09.83**

(54) **Repair of tissue in animals.**

(30) Priority: **24.09.82 US 423203**
       **22.02.83 US 468590**
       **03.06.83 US 500833**

(43) Date of publication of application:
      **04.04.84 Bulletin 84/14**

(45) Publication of the grant of the patent:
      **20.05.92 Bulletin 92/21**

(84) Designated Contracting States:
      **AT BE CH DE FR GB IT LI NL SE**

(56) References cited:
      **EP-A- 0 128 849**

      **Proc. Natl. ACAD. SCI. USA, vol. 80, June 1983, pages 3676-3680; US C.A. Frolik et al.: "Purification and initial characterization of a type beta transforming growth factor from human placenta."**

(73) Proprietor: **THE UNITED STATES OF AMERICA as represented by the Secretary, United States Department of Commerce National Technical Information Service, Office of Government Inventions and Patents, 5285 Port Royal Road Springfield, Virginia 22161(US)**

(72) Inventor: **Sporn, Michael Benjamin 6630 Marywood Road Bethesda, MD 20817(US)**
      Inventor: **Roberts, Anita Bauer 9919 Harrogate Road Bethesda, MD 20817(US)**

(74) Representative: **Strehl, Schübel-Hopf, Groening Maximilianstrasse 54 Postfach 22 14 55 W-8000 München 22(DE)**

EP 0 105 014 B1

Proc. Natl. Acad. Sci. USA, vol. 77, no. 6, June 1980, pages 3494-3498; US; A.B. Roberts et al.: "Transforming growth factors: Isolation of polypeptides from virally and chemically transformed cells by acid/ethanol extraction."

Chemical Abstracts, vol.97, no.17, October 25, 1982, page 93, ref. 138800v; Columbus, Ohio, US M.A. Anzano et al.: "Purification by reverse-phase high-performance liquid chromatography of an epidermal growth factor-dependent transforming growth factor." & ANAL. Biochem., 1982, 125(1), 217-24

Chemical Abstracts, vol.97, no.23, December 6, 1982, page 419, ref. 196472a; Columbus, Ohio, US M.A. Anzano et al.: "Synergistic interaction of two classes of transforming growth factors from murine sarcoma cells." & Cancer Res., 1982, 42(11), 4776-8

Science, vol. 219, March 18, 1983, pages 1329-1331 M.B. Sporn et al.: "Polypeptide transforming growth factors isolated from bovine sources and used for wound healing in vivo."

Proc. Natl. ACAD. SCI. USA; vol. 78, no.9, September 1981, pages 5339-5343; US A.B. Roberts et al.: "New class of transforming growth factors potentiated by epidermal growth factor: Isolation from non-neoplastic tissues."

NATURE, vol. 295, February 4, 1982, pages 417-419; Macmillan Journals Ltd., US A.B. ROBERTS et al.: "Isolation from murine sarcoma cells of novel transforming growth factors potentiated by EGF"

**Description**

This invention relates to a purified $\beta$-type transforming growth factor in the form of a homogeneous protein and to a method for its production as well as to compositions comprising this purified $\beta$-type transforming growth factor, and to the use of such compositions for producing a medicine.

There is a continuing need for the promotion of rapid cell proliferation at the site of wounds, burns, diabetic and decubitus ulcers, and other traumata.

A number of growth factors are known which promote the rapid growth of animal cells. These growth factors include epidermal growth factor (EGF), transforming growth factors (TGF's) and nerve growth factor (NGF).

Many new peptide growth factors have been isolated and characterized recently, as indicated in Tissue Growth Factors, R. Baserga, ed., Springer-Verlag pub., New York (1981), however there have been few studies on the activity of these materials in vivo. In many cases, the relatively small amounts of peptides available have limited the ability to study their properties in vivo. An important area for potential application of peptide growth factors is the enhancement of wound healing. Despite the need for rapid wound healing in the treatment of severe burns, trauma, diabetic and decubitus ulcers, and many other conditions, at present there is no practical way to accelerate wound healing with pharmacological agents. Although it is suggested in Tissue Growth Factors, supra, at p. 123 that EGF might be of benefit in this area, it has yet to be extensively used in a practical way for wound healing.

The term, "transforming growth factor" (TGF) has been defined to include the set of polypeptides which confer the transformed phenotype on untransformed indicator cells. The transformed phenotype is operationally defined by the loss of density-dependent inhibition of growth in monolayer, overgrowth in monolayer, characteristic change in cellular morphology, and acquisition of anchorage independence, with the resultant ability to grow in soft agar. Untransformed, non-neoplastic cells will not form progressively growing colonies in soft agar, while the property of anchorage-independent growth of cells in culture has a particularly high correlation with neoplastic growth in vivo.

Although TGFs were first discovered in the conditioned medium of virally-transformed neoplastic mouse cells, the application of the acid/ethanol method for extraction of peptides from tissues has now shown that TGFs can be found in almost all tissues, both neoplastic and non-neoplastic, from all species of animals that have been examined thus far. Although TGF activity is usually measured with an in vitro phenotypic transformation assay, this does not imply that TGF activity in vivo is necessarily related to the development of malignancy. Indeed, the transformed phenotype is a physiological state associated with normal embryological development, and transforming (onc) genes have been found in normal cells of essentially all vertebrates. The function of these onc genes from normal cells is not known at present. While there may be irreversible and excessive expression of TGF activity during malignant cell growth, the data at hand indicate that TGFs have a more benign and perhaps essential role in the function of normal cells. At present, it is not known what the intrinsic physiological roles of TGFs are. In this respect, TGFs are like many other peptide hormones or hormone-like agents which have recently been discovered and isolated; this is particularly true for many peptides of the nervous system, for which a defined chemical structure may be known, yet whose physiology is still a matter of uncertainty.

The initial description of sarcoma growth factor (SGF), the first of the TGFs to be isolated, was an important finding in tumor cell biology since it provided a direct mechanism for the expression of the neoplastic phenotype in a virally-transformed cell. Two important properties of SGF were described in these earliest studies, namely (1) that the effects of SGF in causing phenotypic transformation were dependent on its continued presence, and that these effects were reversible when SGF was removed, and (2) that the effects of SGF could be expressed in the very same cells that synthesized this peptide, a property that has been termed autocrine secretion. Although these two properties have not been definitively shown for all of the other more newly discovered TGFs, the functions of the entire set of TGFs can most reasonably be assumed to be that of local, hormone-like agents that reversibly control cell function by paracrine or autocrine mechanisms.

Since the discovery of SGF in 1978, many TGFs have been described from diverse sources. These TGFs can be categorized into two groups: extracellular TGFs isolated from conditioned media of cultured cells, and intracellular (cell-associated) TGFs isolated by direct extraction of cells or tissues. Although extracellular TGFs have recently been isolated from non-neoplastic murine cells, use of conditioned medium has, in general, been restricted to neoplastic cell lines that could be grown in long-term, large-scale culture, including certain virally and chemically-transformed rodent cells and human tumor cell lines. The adaptation of an acid/ethanol extraction procedure to TGF isolation removed all limitations on cell types and quantities of tissues that could be examined. Using this procedure, extracts of all tissues or cells, whether of

3

neoplastic or non-neoplastic origin, whether from adult or embryonic tissue, whether from human, bovine, or murine genomes, have been shown to promote colony formation in a soft agar assay; hence, by definition, these extracts contain TGF activity.

A variety of both epithelial and fibroblastic cell lines form colonies in soft agar in the presence of TGFs. However, the most commonly used indicator cell line is the rat kidney fibroblast cell clone, NRK 49F, which has been selected for its strong colony-forming response to the TGFs. Rat-1 cells and mouse AKR-2B cells have also been used successfully as indicator cells.

All of the TGFs referred to above are low molecular weight polypeptides which share with SGF the physical properties of acid and heat stability and sensitivity to treatment with both trypsin and dithiothreitol. However, there are marked differences in the biological properties of these TGFs, particularly with respect to their relationship to EGF. Certain TGFs, though antigenically distinct from EGF, have some structural homology to EGF, since they compete with EGF for receptor binding. Other TGFs do not compete with EGF for receptor binding, but instead are dependent on EGF for activity in the soft agar assay for colony formation. To remove the ambiguities implicit in the assignment of the general term "TGF" to these different factors, an operational classification of the members of the TGF family based on their interactions with EGF is suggested, both with respect to competition for binding to the EGF receptor and to the requirement for EGF for induction of colonies in soft agar.

As defined for purposes of this invention, TGF-$\alpha$ are those TGFs which compete with EGF for receptor binding and which do not require EGF for the induction of colony formation in soft agar. TGFs with these properties include SGF and other TGFs derived from neoplastic cells, as well as some TGFs from mouse embryos.

As defined for purposes of this invention, TGF-$\beta$ are those TGFs which do not compete with EGF for receptor binding and which require EGF for the induction of colony growth in soft agar. When assayed in the presence of EGF, TGF-$\beta$ represents the major colony-forming activity of the intracellular TGFs of both neoplastic and non-neoplastic cell lines and tissues. It can be assumed that TGF-$\beta$ will be found in conditioned media as well, once the proper assays are used.

Those TGFs which do not compete with EGF for receptor binding and which do not require EGF for colony formation are designated TGF-$\gamma$ (gamma-type TGF). Such TGFs have been described in conditioned media of certain virally or chemically transformed cells. Finally, TGF-$\delta$ (delta-type TGF) is used to specify those TGFs which would both compete for EGF receptors and require EGF for colony formation in soft agar. EGF itself could be classified as a weak TGF-$\delta$.

The state of the art shows that there were made several attempts to isolate and purify beta-type transforming growth factors (TGF-$\beta$), nevertheless none of these prior attempts has been useful to produce TGF-$\beta$ in the form of a homogeneous purified protein pure enough to be usable for preparing a pharmaceutical composition.

The article by A. B. Roberts et al in Proc. Natl. Acad. Sci. USA, vol. 78, No. 9, September 1981, pages 5339-5343 "New class of transforming growth factors potentiated by epidermal growth factor: Isolation from non-neoplastic tissues" describes the production of a protein preparation which has a purification degree of the 2,200 fold of the crude extract and about the 22,000 fold based on the original tissues.

The article by A. B. Roberts et al in Nature, vol. 295, February 4, 1982, pages 417-419; Macmillan Journals Ltd. "Isolation from murine sarcoma cells of novel transforming growth factors potentiated by EGF" describes a purification method similar to the method of the above-mentioned reference which is used for isolating two different growth factors, i.e. TGF-$\alpha$ and TGF-$\beta$, from murine sarcoma cells. In this case too, no purified homogeneous protein could be obtained, it was rather only possible to obtain two different fractions of the two different transformation growth factors.

The article by A. B. Roberts et al in Proc. Natl. Acad. Sci. USA, vol. 77, No. 6, June 1980, pages 3494-3498 "Transforming growth factors: Isolation of polypeptides from virally and chemically transformed cells by acid/ethanol extraction" also describes the isolation of two different polypeptide fractions having an apparent molecular weight of 7,000 and 10,000 from murine sarcoma. From the statements at page 3494, left hand column, first part (abstract) and the discussion at page 3498, it may be seen that the authors of this article admit that they could not obtain a purified product: "proof of the chemical and structural relatedness of these peptides must await further purification..." (page 3498, left hand column, second paragraph). Similarly, the abstract of this article shows that only a 22,000 fold purification could be achieved.

An article by M.A. Anzano et al in Anal. Biochem. 1982, 125(1), pages 217-24 confirms the fact that all prior art methods resulted in a certain purification but were, however, not able to isolate a homogeneous protein. According to said article the last purification step of the method described there resulted in only a 430 fold purification.

The latter reference is based on experiments carried out by the inventors of the present patent application. It was found only later by the present inventors that the preparation described there was not TGF-$\beta$ and that the stated reference does not provide a teaching enabling the average skilled man to isolate TGF-$\beta$ in the form of a homogeneous, purified protein.

Considering the above described state of the art it is the objective of the present invention to provide a $\beta$-type transforming growth factor in extremely high purity sufficient for the pharmaceutical use.

The subject-matter of the present invention is a purified $\beta$-type transforming growth factor in the form of a homogeneous protein, susceptible to Edman-degradative amino-acid sequencing which is obtainable by the steps of extracting murine, human or bovine cells or tissues with acid-ethanol to produce a cell extract, dialysing the cell extract to produce a dialysed acid-ethanol extract, applying the dialysed acid-ethanol extract to a gel-filtration chromotography column and eluting with 1M acetic acid to obtain a gel filtration eluate, adding the gel-filtration eluate to a reverse-phase HPLC $C_{18}$ column using an acetonitrile gradient in 0.1% triflucroacetic acid to produce a $C_{18}$ eluate, applying the $C_{18}$ eluate to a CN reverse-phase HPLC column using a n-propanol gradient in 0.1% trifluoroacetic acid to obtain a CN eluate, and recovering from the CN-eluate a sequencible TGF-$\beta$ that runs as a 12,500-13,000 dalton molecular weight protein on a reducing SDS-PAGE gel and a 25,000-26,000 dalton molecular weight protein on a non-reducing SDS-PAGE gel.

The present invention furthermore concerns a process for preparing the said purified beta-type transforming growth factor, comprising the above described process steps.

This invention further provides a composition for the promotion of cell proliferation and tissue repair whose active ingredient consists essentially of the above defined $\beta$-type transforming growth factor (TGF-$\beta$) in amounts sufficient to promote cell proliferation.

Preferably such composition further comprises as an additional ingredient at least one activating agent present in an amount sufficient to activate said TGF-$\beta$, wherein the activating agent is selected from the group consisting of epidermal growth factor (EGF) and $\alpha$-type transforming growth factor (TGF-$\alpha$). Active ingredients are present in an amount sufficient to promote cell proliferation.

The present invention furthermore comprises the use of the above stated composition for the production of a medicine having a utility in the treatment of mammalian systemic traumata by systemic administration.

In the compositions the TGF-$\beta$ and the activating agent are preferably present in about equimolar amounts, and the active ingredients are present in an amount at least sufficient to stimulate cell proliferation (tissue repair).

As another embodiment, the activated TGF-$\beta$ compositions of this invention may be admixed with other (secondary) growth factors to enhance their activity.

The compositions may be formulated in any suitable carrier for topical application, such as physiological saline solution or purified collagen suspension. The compositions also may be formulated in any suitable carrier for systemic administration.

The method of topical administration of the compositions of this invention is by direct application to a burn, wound, or other traumata situs. Periodic or continual further administration may be preferably indicated in most instances, since the active ingredients are physiologically utilized by the cells whose growth is being stimulated.

As a further embodiment, the compositions of this invention may be administered systemically by injection, enterally, transdermal patches, and the like, depending upon the nature and site of the traumata to be treated.

## Example Of Purification And Properties

Research in our laboratory has been directed toward the isolation of TGFs directly from cells and tissues. An acid/ethanol extraction procedure was modified for this purpose, as disclosed in A. B. Roberts, et al., Proc. Natl. Acad. Sci., U.S.A., 77:3494-3498 (1980). In more detail according to this process cells or tissues are thawed in 40ml/10g of tissue of a solution consisting of 375ml of 95% (vol/vol) ethanol and 7.5ml of concentrated HCl, plus 33mg of phenylmethylsulfonylfluoride and 1.9ml of pepstatin as protease inhibitors. The volume is adjusted with distilled water 60ml/10g of tissue and the tissue is minced. After overnight extraction at 4°C the mixture is centrifuged and the residue is reextracted for 2hrs with 40ml of a solution of 375ml of 95% ethanol, 105ml of distilled water and 7.5ml of concentrated HCl. The combined supernatants are kept in the acidic range by adjustment to pH 5.2 with concentrated ammonium hydroxide followed by the addition of 1ml of 2 M ammonium acetate buffer, pH 5.3 per 85ml of extract. Two volumes of cold anhydrous ethanol and four volumes of cold anhydrous ether are immediately added, whereafter the mixture is allowed to stand at -20°C for 30-48 hours. The resulting precipitate is collected by centrifugation or by rapid filtration through Whatman no. 1 paper and redissolved in 1 M acetic acid (3-4ml per gram of tissue). The acetic acid-insoluble residue (10-25% of the total solvent-precipitated protein) which has little or no activity in the soft agar assay is discarded. After extensive dialysis at 4°C against 0.17 M acetic acid (Spectropor tubing, molecular weight cutoff 3500, Spectrum Medical Industries ) the samples are lyophilized to dryness. The obtained samples may be redissolved in 1 M acetic acid before being subjected to chromatography. After that chromatography and high pressure liquid chromatography (HPLC) have been employed for further purification. TGF activity, measured by the ability to induce non-neoplastic indicator cells (NRK) to form colonies in soft agar, has been quantitated on an image analysis system with respect to both the number and size of the colonies formed. By use of HPLC, we have shown that two distinctly different TGFs, here classified as TGF-$\alpha$ and TGF-$\beta$, can be isolated from the same pool of acid/ethanol extracts of MSV-transformed 3T3 cells; for this purpose, columns using a linear gradient of acetonitrile in 0.1% trifluoroacetic acid have been used. TGF-$\alpha$, which elutes from the column earlier than marker EGF, is characterized by its ability to induce the formation of small colonies (850-3,100 $\mu m^2$) in soft agar in the absence of added EGF and its ability to compete with EGF in a radio-receptor assay. TGF-$\beta$, which elutes later than TGF-$\alpha$ or marker EGF, does not compete with EGF for receptor binding and requires EGF to induce the formation of large colonies (>3,100 $\mu m^2$) in the soft agar assay.

TGF-$\alpha$ from MSV-transformed 3T3 cells resembles SGF isolated from the conditioned medium of the same cells and other TGFs isolated from rat and human tumor cell lines. Recently, SGF and the TGF-$\alpha$'s from the conditioned media of a human melanoma cell line and virally-transformed rat embryo fibroblast have been purified to homogeneity. The human melanoma TGF-$\alpha$ is a single chain polypeptide of molecular weight 7,400. Its amino acid composition and chromatographic behavior are markedly different from that of human EGF, but similar to that of murine SGF and rat TGF-$\alpha$, suggesting that TGF-$\alpha$'s from human, rat and mouse genomes are more closely related to each other than to EGF. There is therefore a reasonable possibility that TGF-$\alpha$'s may have cross-species utility.

In sarcoma virus-transformed rodent cell lines, the release of TGF-$\alpha$ into the medium has been correlated with the expression of the transformed phenotype, and within a selected set of human tumor cell lines that release TGF-$\alpha$, the ability of the tumor cells to grow in soft agar has been correlated with the quantity of TGF-$\alpha$ they secrete. However, secretion of TGF-$\alpha$ is not an absolute requirement for neoplastic behavior; certain chemically transformed murine cell lines and human lung carcinoma cell lines that do not secrete TGF-$\alpha$ release strong TGF activity that does not compete with EGF for receptor binding.

TGF-$\beta$ of the acid/ethanol extract of the MSV-transformed 3T3 cells resembles other TGFs isolated from many neoplastic and non-neoplastic tissues. After further purification on a second HPLC column, TGF-$\beta$ of the MSV-transformed cells eluted at the same position in the n-propanol gradient (48%) as one peak of TGF-$\beta$ activity of the bovine salivary gland, and each was associated with a small peak of absorbance at 280 nm. These two TGF-$\beta$'s, one from a neoplastic mouse cell line and the other from a non-neoplastic bovine tissue, each migrated as a 12,500-13,000 daltons MW protein on SDS-PAGE in the presence of mercaptoethanol and as an apparent 25,000-26,000 daltons protein in the absence of mercaptoethanol; they therefore appear to be closely related to each other and different from both TGF-$\alpha$ and EGF. The finding of TGF-$\beta$ in all non-neoplastic tissues examined thus far suggests a normal physiological function for these TGFs. There is therefore a reasonable possibility that TGF-$\beta$'s may have cross-species utility.

## Amino Acid Composition and Sequencing of TGF-$\beta$

Through a combination of techniques, TGF-$\beta$ from bovine kidneys was purified 200,000-fold to the point of homogeniety.

Samples of homogenous TGF-$\beta$ (20-50 pmol) were taken to dryness and hydrolyzed in sealed, evacuated tubes at 150°C for 2 hours in 100 $\mu l$ constant boiling HCl containing 0.1% liquid phenol. Half-cystine and methionine were determined by performic acid oxidation followed by acid hydrolysis. Analyses of o-phthalaldehyde derivatives of the amino acids were done on a modified amino acid analyzer equipped with a fluorometer and computing integrator.

For amino-terminal sequence analysis, approximately 500 picomoles ($M_r$ 25,000) of TGF-$\beta$ were reduced and S-carboxymethylated with dithiothreitol and iodo-[$^{14}$C] acetic acid in the presence of 6M guanidine-HCl in 1 M Tris-HCl buffer, pH 8.4. Excess reagents were separated from carboxymethylated protein by HPLC on a 5 micron 50 x 4.6 mm column eluted with a gradient of 0-90% acetonitrile (1% per min) in 0.1% TFA. Overall recovery of the procedure was 96%, based on estimating the amount of protein by amino acid analysis using fluorescamine detection.

Automated Edman degradation was performed on about 500 pmoles ($M_r$ 12,500) of the S-carboxymethylated protein with a gas-phase sequencer. PTH-amino acids were identified using an HPLC system. Initial yeild was about 30% and repetitive yield about 90%.

The following table summarizes the amino acid distribution.

TABLE 1

Amino Acid Composition of Bovine Kidney TGF-β [a]

| Amino Acid | Residues/mole [b] (mean ± range) |
|---|---|
| Aspartic acid | 25 ± 1 |
| Threonine | 7 ± 1 |
| Serine | 16 ± 2 |
| Glutamic acid | 26 ± 1 |
| Proline | N.D. |
| Glycine | 15 ± 3 |
| Alanine | 18 ± 1 |
| Half-cystine [c] | 16 ± 2 |
| Valine | 17 ± 1 |
| Methionine [c] | 3 ± 0 |
| Isoleucine | 11 ± 1 |
| Leucine | 25 ± 1 |
| Tyrosine | 17 ± 2 |
| Phenylalanine | 8 ± 1 |
| Histidine | 8 ± 1 |
| Lysine | 22 ± 2 |
| Tryptophan | N.D. |
| Arginine | 12 ± 1 |

[a]Amino acid composition of purified bovine kidney type β TGF determined after 2 h hydrolysis in 6 N HCl at 150°. Values are based on 3 separate determinations of 3 different preparations.

[b]The number of residues per mole for each amino acid is based on an apparent molecular weight of 25,000.

[c]Determined by performic acid oxidation and acid hydrolysis.

N.D. – not determined

Analysis of the bovine kidney TGF-$\beta$ by electrophoresis on NaDodSO$_4$-polyacrylamide gels suggests that some of the disulfide bonds are inter-chain. Comparison of the above results with those obtained by the analysis of TGF-$\beta$ from human placenta or human platelets revealed no significant differences in the respective amino acid compositions, and it is anticipated that the N-terminal sequences will be very similar.

Amino acid sequence analysis of the reduced and S-carboxymethylated bovine kidney TGF-$\beta$ by automated Edman degradation using a gas-phase sequencer revealed a single N-terminal amino acid sequence as follows, (CMC is S-carboxymethylcysteine):

Ala-Leu-Asp-Thr-Asn-Tyr-CMC-Phe-Ser-Ser-Thr-Glu-Lys-Asn-CMC-.

Initial and repetitive yields were found to be equal to the yields calculated for myoglobin used as standard protein. At the minimum, the results indicate that the sequence of at least the first fifteen N-terminal amino acids of each of the two subunits of TGF-$\beta$ is identical and confirm the observations of a single protein band of the reduced TGF-$\beta$ on NaDodSO$_4$-polyacrylamide gels. In addition, the N-terminal sequence of the bovine kidney TGF-$\beta$ is identical to the partial sequence of TGF-$\beta$ from human placenta, suggesting a high degree of relatedness of type $\beta$ TGFs from different species and different tissue sources.

Activation of TGF-$\beta$

Recent experiments in our laboratory have shown that either TGF-$\alpha$ or EGF will activate TGF-$\beta$ to induce the formation of large colonies in soft agar. Purified TGF-$\beta$ from the MSV-transformed 3T3 cells, assayed by itself, had no colony-forming activity at concentrations as high as 2 $\mu$g/ml. However, assayed after activation by the presence of either EGF, or TGF-$\alpha$ derived from the same cells, TGF-$\beta$ induced a dose-dependent formation of large colonies (>3,100 $\mu$m$^2$) at concentrations of 10-200 ng/ml. By contrast, EGF or TGF-$\alpha$, assayed by themselves, induced a maximal response of only a small number of colonies; this response was increased 10-fold by the addition of TGF-$\beta$. The relative abilities of EGF and TGF-$\alpha$ to promote TGF-$\beta$-dependent formation of large colonies in soft agar correlated with their relative abilities to compete for binding to the EGF receptor; other experiments using chemically-modified EGF analogues have substantiated this correlation. These data, demonstrating that the induction of a strong colony-forming response requires both TGF-$\alpha$ and TGF-$\beta$ or EGF, suggest that TGF-$\beta$, which is found in all tissues, may be an essential mediator of the effects of TGF-$\alpha$ and of EGF on neoplastic transformation.

Little is known about the mechanisms by which exogenous TGFs induce non-neoplastic cells to express the transformed phenotype. Furthermore, the synergistic interactions of TGF-$\alpha$ and TGF-$\beta$ suggest that these two TGFs may act through different pathways. Experiments using TGFs of conditioned media of sarcoma virus-transformed rodent cells have shown that the synthesis of new RNA and protein is required before transformation occurs. Other experiments have been directed at a possible role of TGFs in phosphorylation reactions. Certain viral transforming gene products and their normal cellular homologues have tyrosine-specific protein kinase activity, and it has been proposed that phosphorylation at tyrosine of specific substrates may be important in the transformation process. Treatment of human carcinoma A431 cells with various TGFs derived from conditioned media of virally-transformed cells or human tumor cell

lines (TGF-$\alpha$) resulted in phosphorylation of tyrosine residues in the 160 K EGF receptor. The pattern of phosphorylation, however, was indistinguishable from that induced by EGF itself, and thus would not appear to be transformation-specific. Likewise, dissolution of actin fibers of Rat-1 cells occurs when they are treated with either TGF or EGF. It is clear that further research is needed to establish the relationships of the TGFs to the retrovirus transforming gene products and the mode of action of the TGFs in neoplastic transformation.

The following are summaries of examples which illustrate various aspects of this invention:

Example 1.

HPLC separation of TGF-$\alpha$ and TGF-$\beta$ of MSV-transformed 3T3 cells. The acid/ethanol extract of MSV-transformed cells was chromatographed on Bio-Gel P-30 in 1 M acetic acid. The 7-10,000 MW TGF fraction was further purified on a $\mu$Bondapak C18 column using a gradient of acetonitrile in 0.1% trifluoroacetic acid. Aliquots were assayed for colony-forming activity in the soft agar assay; in the presence of 2 ng/ml EGF; and in competition with $^{125}$I-EGF in a radio-receptor assay.

Example 2.

HPLC purification on $\mu$Bondapak CN columns of TGF-$\beta$ from MSV-transformed mouse 3T3 cells and bovine salivary gland using a gradient of n-propanol in 0.1% trifluoroacetic acid. TGF-$\beta$ of acid/ethanol extracts was purified on Bio-Gel P-30 and $\mu$Bondapak C18 columns and then applied to CN columns. Aliquots were assayed for induction of colony growth of NRK cells in soft agar in the presence of 2 ng/ml EGF.

Example 3.

Synergistic interaction (activation) of TGF-$\beta$ with TGF-$\alpha$ to induce the formation of large colonies of NRK cells in soft agar. Soft agar colony-forming activity of varying concentrations of $\mu$Bondapak CN-purified TGF-$\beta$ derived from MSV-transformed 3T3 cells was assayed either alone or in the presence of either CN-purified TGF-$\alpha$ derived from the same cells or murine EGF. Soft agar colony-forming activity of varying concentrations of EGF or TGF-$\alpha$ was assayed either alone or in the presence of TGF-$\beta$.

In Vivo Demonstration Of Wound Healing

After the above in vitro demonstrations of the operability of the compositions of this invention, it was considered critical to confirm that the compositions could work in clinical applications. For this purpose, TGFs were isolated on a relatively large scale from bovine sources and the wound healing activity of the compositions according to this invention were satisfactorily demonstrated using an experimental rodent wound healing protocol.

The examples which follow demonstrate not only that the compositions according to this invention are effective in vivo, but also that TGFs may be employed cross-species.

Example 4.

Purification and separation of TGF-$\alpha$ and TGF-$\beta$.Bovine tissues, obtained fresh from the slaughterhouse and frozen immediately on dry ice, were extracted in 2kg batches with acid/ethanol in accordance with the above described process of A. B. Roberts, et al., Proc. Natl. Acad. Sci., U.S.A., 77:3494 (1980). Extracts from 6-8 kg tissue were combined and chromatographed on Bio-Gel P-30 with 1 M acetic acid, using an 80 liter bed volume column. The TGFs of extracts of bovine kidney or bovine salivary gland eluted in a broad peak between the RNase (13,700) and insulin (5,700) markers, as had been observed for the TGFs of mouse kidney and mouse salivary gland. TGFs at this stage of purification had a specific activity approximately 10 to 25-fold higher than the acid/ethanol extracts, with a range of recovery of 150,000-200,000 colony-forming units per kg tissue. Most of the in vivo studies reported below were done with salivary gland or kidney TGFS purified to this stage. The TGFs activity in vitro was enhanced approximately 20-fold by the presence of 2-5 ng EGF per ml in the asssay, in accordance with this invention.

Following chromatography on Bio-Gel P-30, the bovine TGF-$\beta$ were purified further by High Pressure Liquid Chromatography (HPLC) on $\mu$Bondapak C18 columns using an acetonitrile gradient in 0.1 percent trifluoroacetic acid, followed by a second HPLC step on $\mu$Bondapak CN columns using a gradient of n-propanol in 0.1 percent trifluoroacetic acid. After the two HPLC steps, analysis of the bovine TGF-βs from both salivary gland and kidney by sodium dodecyl sulfate polyacrylamide gel electrophoresis under reducing conditions showed a single band with an apparent molecular weight of 13,000 daltons. At this stage of purification, each of the bovine TGF-β's had an absolute requirement for EGF for colony-forming activity. The yield of HPLC-purified TGF-β was approximately 20-100 μg per kg tissue, with a total activity of 7,000-18,000 colony-forming units.

Example 5.

Wound healing protocol. In vivo activity of isolated salivary gland TGF-β and kidney TGF-β was measured in accordance with the protocol described by T. K. Hunt, et al., Amer. J. Surgery, 114:302 (1967). Six empty Schilling-Hunt wire mesh wound chambers were surgically inserted subcutaneously in the backs of rats, in paired symmetrical fashion (A-D, B-E, C-F) as shown below:

TABLE 2

head

| A | D |
|---|---|
| B | E |
| C | F |

tail

The rats respond to these chambers as if they were wounds, and eventually the chambers become filled with fibroblasts and collagen. By the fourth day after insertion, the chambers become encapsulated with connective tissue, but there are few cells within the chambers themselves. There is thus a defined, enclosed space within the chambers, where a wound healing response can be quantitatively measured. At this time, daily injections of TGF-β (0.1 ml, in sterile phosphate-buffered saline)into chambers A, B, and C were begun. To activate TGF-β activity, a low level of murine EGF was included in all TGF-β injections, unless noted otherwise. Chambers D, E, and F were used as controls, and were injected with either an amount of bovine serum albumin (BSA) alone or in combination with either TGF-β or EGF, such that the total protein was equivalent to the amount of TGF-β injected into chambers A, B, and C. Injections were made once daily for either 5 days (Table 2) or 9 days (Table 3). All injected materials were sterile. The rats were sacrificed 6 hours after the last TGF-β injection; in Table 3 they were injected with 0.5 mCi of thymidine-$^3$H, specific activity 6.7 Ci/millimole (i.p.) together with the last TGF-β injection. The chambers were removed from the rats, all connective tissue on the outside of the wire mesh was peeled away, and then the contents of each chamber were determined.

Table 3. Wound healing response to bovine salivary gland or kidney TGF after 5 days of treatment. TGF-βs were prepared and injected as described. Each dose contained 25 times the amount of TGF-β found optimal for colony formation by NRK cells in a standard soft agar assay, and ranged from 18-42 colony forming units per dose. The amounts of protein injected per dose were: 7 µg in Expts. 1, 4 and 5; 25 µg in Expt. 3, and 0.7 µg in Expt 2. All doses of EGF were 20 ng. Total protein in wound chambers was measured by the method of Lowry, et al.** Statistical analysis of the data was made by comparison of matched pairs of the chambers (A vs. D, B vs. E, C vs. F) shown in Table 2.

| Expt. | Chamber A,B,C treatment | Chamber D,E,F treatment | Number of matched pairs of chambers | Milligrams of protein per chamber average A,B,C | D,E,F | Average ratio ± standard error of mean* | †† |
|---|---|---|---|---|---|---|---|
| 1 | TGF-β (Salivary P-30) + EGF | BSA | 36 | 10 | 3.9 | 3.8 ± 0.6 | <0.001 |
| 2 | TGF-β (HPLC) + EGF | BSA | 9 | 8.4 | 2.9 | 4.6 ± 1.0 | <0.02 |
| 3 | TGF-β (Kidney P-30) + EGF | BSA | 9 | 8.1 | 3.5 | 5.2 ± 1.5 | <0.005 |
| 4 | TGF-β P-30) + EGF | EGF | 9 | 9.6 | 5.3 | 2.1 ± 0.3 | <0.02 |
| 5 | TGF-β (Salivary P-30) + EGF | TGF-β | 9 | 11.2 | 9.6 | 1.4 ± 0.3 | 0.5 |

*Average of each matched pair ratio, A/D, B/E, C/F

**J. Biol. Chem, 193:265 (1951)

†One sided P values based on the sign test

12

Table 4. Wound healing response to bovine salivary gland TGF-β after 9 days of treatment. Chambers A, B, and C were dosed once daily with 7 μg of TGF-β (P-30) plus 20 ng of EGF. Chambers D, E and F were dosed with an equal amount of BSA.

| Measurement | Number of matched pairs of chambers | Average content per chamber A,B,C | Average content per chamber D,E,F | Average ratio ± standard error of mean* | P† |
|---|---|---|---|---|---|
| Protein, milligrams | 30 | 24 | 15 | 1.6 ± 0.05 | <0.001 |
| DNA, micrograms | 30 | 21 | 8.6 | 2.6 ± 0.16 | <0.001 |
| Thymidine-³H, cpm per microgram of DNA | 30 | 45 | 30 | 1.7 ± 0.09 | <0.001 |
| Collagen, milligrams | 9 | 5.2 | 3.2 | 1.8 ± 0.2 | <0.005 |

*Average of each matched ratio pair, A/D, B/E, C/F

†One sided P values based on the sign test

Table 3 shows that 5 days of treatment of rats with TGF-β from either bovine salivary gland or bovine kidney caused a significant increase in total protein in the treated chambers, as compared to control chambers treated with an equivalent amount of bovine serum albumin (Experiments 1, 3). The salivary gland TGF-β was still highly active after two steps of purification by the high pressure liquid chromatography (Experiment 2). The effects observed are not the sole result of the minute amounts of EGF which had been used to potentiate the activity of TGF-β, since a highly significant difference between treated

13

chambers A, B and C, compared to control chambers D, E and F was still observed when EGF was used as the control substance (Experiment 4). Furthermore, when all chambers were treated with TGF-$\beta$, and only A, B, and C were treated with EGF, no significant difference was observed (Experiment 5). At the end of Experiments 1-4, it was consistently observed that chambers A, B and C were more firmly fixed in the surrounding connective tissue than the respective matched control chambers, suggesting that effects of the TGF-$\beta$ also were manifested in the area immediately surrounding the chambers.

In order to measure the effects of bovine salivary TGF-$\beta$ on DNA and collagen content of the chambers, it was necessary to treat the animals for longer than 5 days. Table 4 shows the results of a larger experiment in which 13 rats were treated for 9 days. The increases in total protein, total DNA, thymidine incorporation into DNA, and total collagen were all highly sufficient. Histological examination of the contents of the chambers treated with TGF-$\beta$ confirmed the occurrence of fibroblastic proliferation and formation of collagen. A sterile infiltrate of inflammatory cells was also found within both treated and control chambers.

The results obtained in both experiments indicate that TGF-$\beta$s when activated in accordance with this invention, can significantly accelerate a wound healing response.

Clinical Use Of The Compositions Of This Invention

The compositions of this invention, whose active ingredients are TGF-$\beta$ activated by at least one of a TGF-$\alpha$ and an EGF, can reasonably be expected to have clinical use in the treatment of animals, particularly mammals, most particularly human beings. There are several sound bases for this conclusion.

It has been shown above, that in in vitro tests, the compositions can markedly increase the growth of cells without changing their genotype. An important characteristic of the components of the compositions of this invention, is that they do not appear to be species specific. That is, TGF-$\beta$ from one species can be activated by TGF-$\alpha$ and/or EGF from other species. The cells whose growth is promoted can be of any type such as fibroblast or epithelial, although it is considered that the growth promotion of fibroblast cells will have the greatest medical utility.

The in vivo experimental protocol disclosed above, with its very favorable results, clearly indicates that the compositions of this invention have utility in the treatment of traumata by the rapid promotion of the proliferation of the cells surrounding the traumata.

Two types of application of the compositions of this invention are contemplated.

The first, and preferred, application is topically for the promotion of surface wound healing. There are no limitations as to the type of wound or other traumata that can be treated, and these include (but are not limited to): first, second and third degree burns (especially second and third degree); surgical incisions, including those of cosmetic surgery; wounds, including lacerations, incisions, and penetrations; and surface ulcers including decubital (bed-sores), diabetic, dental, haemophiliac, and varicose. Although the primary concern is the healing of major wounds by fibroblast cell regeneration, it is contemplated that the compositions may also be useful for minor wounds, and for cosmetic regeneration of cells such as epithelial. It is also contemplated that the compositions may be utilized by the topical application to internal surgical incisions.

When applied topically, the compositions may be combined with other ingredients, such as carriers and/or adjuvants. There are no limitations on the nature of such other ingredients, except that they must be pharmaceutically acceptable, efficacious for their intended administration, and cannot degrade the activity of the active ingredients of the compositions. When the compositions of this invention are applied to burns, they may be in the form of an irrigant, preferably in combination with physiological saline solution. The compositions can also be in the form of ointments or suspensions, preferably in combination with purified collagen. The compositions also may be impregnated into transdermal patches, plasters, and bandages, preferably in a liquid or semi-liquid form.

The second application is systemically for the healing of internal wounds and similar traumata. Such an application is useful provided that there are no, or limited, undesirable side-effects, such as the stimulation of neoplastic cellular growth.

When applied systemically, the compositions may be formulated as liquids, pills, tablets, lozonges, or the like, for enteral administration, or in liquid form for parenteral injection. The active ingredients may be combined with other ingredients such as carriers and/or adjuvants. There are no limitations on the nature of such other ingredients, except that they must be pharmaceutically acceptable, efficacious for their intended administration, and cannot degrade the activity of the active ingredients of the compositions.

The amount of activating agent (TGF-$\alpha$s or EGFs) present depends directly upon the amount of TGF-$\beta$s present in the activated compositions of this invention. There are indications that the activation is not catalytic in nature, and that therefore approximately stoichiometric (equimolar) quantities are preferred.

The amount of activated composition to be used in the methods of this invention cannot be stated because of the nature of the activity of TGFs and the nature of healing wounds and/or other traumata. As indicated above, the TGFs activate cells by binding to receptor sites on the cells, after which the TGFs are absorbed and utilized by the cells for the synthesis of new protein, resulting in cell multiplication. Thus, the TGFs are consumed by the cell regenerating process itself, rather than acting in an enzymatic or other catalytic manner. Receptors for EGFs have been found on a wide variety of fibroblastic, epithelial, and parietal cells, as disclosed in Gonzalez, et al., J. Cell. Biol., 88:108-144 (1981). Further, it has been calculated that there are 3,000 EGF binding (receptor) sites for each rat intestinal epithelial cell, as disclosed in M. E. Lafitte, et al., FEBS Lett., 114(2):243-246 (1980). It must also be obvious that the amount of a cell growth promoting substance (such as the compositions of this invention) that must be utilized will vary with the size of the wound or other traumata to be treated.

Since the compositions of this invention both provoke and sustain cellular regeneration, a continual application or periodic reapplication of the compositions in indicated.

The amount of active ingredient per unit volume of combined medication for administration is also very difficult to specify, because it depends upon the amount of active ingredients that are afforded directly to the regenerating cells of the wound or other traumata situs. However, it can generally be stated that the TGF-$\beta$s should preferably be present in an amount of at least about 1.0 nanogram per milliliter of combined composition, more preferably in an amount up to about 1.0 milligram per milliliter.

Additional Embodiments Utilizing the Compositions of this Invention

In addition to utilizing the activated TGF-$\beta$ compositions of this invention by themselves, it is possible to use them in combination with secondary growth factors.

The activated transforming growth factors of this invention may be physically admixed with one or more of many other (secondary) peptide and non-peptide growth factors. Such admixtures may be administered in the same manner and for the same purposes as the activated transforming growth factors of this invention utilized alone, to enhance their activity in promoting cell proliferation and repair.

The useful proportions of activated transforming growth factor to secondary growth factors are 1:.1-10 mols, with about equimolar amounts being preferred.

The secondary growth factors may be used alone or in any physiologically and pharmaceutically compatible combination.

The known secondary growth factors, in approximately descending order of usefulness in this invention (by group), include:
1. platelet-derived growth factors
2. fibroblast growth factors
angiogenesis factors
3. insulin-like growth factors including somatomedins
4. insulin
nerve growth factors
5. anabolic steroids.

In addition to the above known secondary growth factors, it is reasonable to expect that as yet undiscovered secondary growth factors will be useful in admixture.

This invention also incorporates the inactive intermediate substance TGF-$\beta$ per se. Prior to this invention, this substance had not been isolated or identified. TGF-$\beta$ is believed to be substantially the same or very similar for each animal species, regardless of the individual of that species or the particular body cells from which it is derived. Since TGF-$\beta$ has been shown to be non-species-specific between rodents, cattle, and human beings, it is also reasonable to believe that the substance is substantially the same or very similar when derived from any mammal, and possibly from any animal source. It should be noted, moreover, that this invention includes TGF-$\beta$ regardless of the source from which it is isolated or derived, including genetically engineered cells. It is well within the capabilities of biochemical technology to genetically engineer a cell to produce TGF-$\beta$ at the present time.

Administration of Unactivated TGF-$\beta$

It is believed that TGF-$\beta$ has no wound-healing or other tissue-repair activity unless it has been activated by an agent as described above.

However, it is noted that Table 3 Experiment 5, supra, appears to indicate statistically similar results for TGF-β activated with EGF (chambers A, B, C) and $\overline{\text{TGF-}\beta}$ per se (chambers D, E, F). The most logical explanation for this, is that the TGF-β per se was activated by $\overline{\text{a}}$ TGF already present in the test animal. Various TGFs, such as EGF, are known to be present in blood plasma.

Thus, the results of Experiment 5 are not inconsistent with this invention, but instead constitute a variant embodiment thereof. Specifically, TGF-β per se may be administered, in accordance with this invention, instead of activated TGF-β, when there are sufficient endogenous activating agents present in an animal, to activate an amount of TGF-β sufficient to promote cell proliferation and tissue repair. It is anticipated that in an animal suffering from the traumata contemplated herein, there usually will not be sufficient endogenous activating agents present.

**Claims**

1. A purified beta-type transforming growth factor in the form of a homogeneous protein, susceptible to Edman-degradative amino-acid sequencing, obtainable by the steps of
   a) extracting murine, human or bovine cells or tissues with acid-ethanol to produce a cell extract;
   b) dialysing the cell extract to produce a dialysed acid-ethanol extract;
   c) applying the dialysed acid-ethanol extract to a gel-filtration chromatography column and eluting with 1M acetic acid to obtain a gel-filtration eluate;
   d) adding the gel-filitration eluate to a reverse-phase HPLC $C_{18}$ column using an acetonitrile gradient in 0.1% trifluoroacetic acid to produce a $C_{18}$ eluate;
   e) applying the $C_{18}$ eluate to a CN reverse-phase HPLC column using a n-propanol gradient in 0.1% trifluoroacetic acid to obtain a CN eluate; and
   f) recovering from the CN-eluate a sequencible TGF-β that runs as a 12,500-13,000 dalton molecular weight protein on a reducing SDS-PAGE gel and a 25,000-26,000 dalton molecular weight protein on a non-reducing SDS-PAGE gel.

2. The growth factor of claim 1 derived from a genetically engineered cell.

3. The growth factor of claim 1 derived from a bovine kidney.

4. The transforming growth factor of any of the claims 1 to 3 having a partial amino acid sequence as determined by Edman Degradation consisting of the following for each of its two subunits:

$$\overset{5}{\phantom{.}}\qquad\qquad\overset{10}{\phantom{.}}\qquad\qquad\overset{15}{\phantom{.}}$$
$$\text{Ala-Leu-Asp-Thr-Asn-Tyr-CMC-Phe-Ser-Ser-Thr-Glu-Lys-Asn-CMC-,}$$

where CMC is Half-cystine or cysteine, determined as S-carboxymethylcysteine.

5. A process for preparing the purified sequencible TGF-β derived from a murine, human or bovine cell or tissue, comprising the steps of claim 1.

6. A composition for the promotion of cell proliferation and tissue repair in animals and humans whose active ingredient consists essentially of a β-type transforming growth factor (TGF-β) according to any of claims 1 to 4 in amounts sufficient to promote cell proliferation.

7. The composition of claim 6, wherein said TGF-β is present in an amount from 1.0 nanogram to 1 milligram per milliliter of the composition.

8. The composition of claims 6 or 7, further containing at least one pharmaceutically acceptable carrier.

9. The composition of claim 8, wherein the carrier is a purified collagen and the composition is a suspension for topical application.

10. The composition of claim 8, wherein the carrier is a physiological saline solution and the composition is an irrigant.

16

11. The composition of any of claims 6 to 10, further comprising as an active ingredient at least one activating agent present in an amount sufficient to activate said TFG-$\beta$, the activating agent being selected from the group consisting of epidermal growth factors (EGF) and alpha-type transforming growth factors (TGF-$\alpha$), which active ingredients are present in an amount sufficient to promote cell proliferation.

12. The composition of claim 11, used for the topical promotion of fibroblast cell proliferation in mammals wherein the TGF-$\beta$ and at least one activating agent are present in approximately equimolar amounts

13. The composition of claim 12, wherein the activating agent is an EGF or a TGF-$\alpha$.

14. The composition of claim 11, wherein the activated TGF-$\beta$ is admixed with at least one secondary growth factor in a mole ratio of 1:0.1-10, preferably in about equimolar amounts.

15. The composition of claim 11 wherein the secondary growth factor is at least one of the group consisting of: platelet-derived growth factors, fibroblast growth factors, angiogenesis growth factors, insulin-like growth factors, insulin, nerve growth factors, and anabolic steroids.

16. Use of a composition according to any of the claims 6 to 15 for the production of a medicine for the treatment of mammalian topical wounds, burns, ulcers and other traumata by topical application.

17. The use of claim 16, wherein the medicine is designed for periodic or continual topical readministration.

18. Use of a composition according to any of the claims 6 to 15 for the production of a medicine having utility in the treatment of mammalian systemic traumata by systemic administration.

19. The use of claim 18, wherein the medicine is designed for periodic or continual systemic readministration.

**Revendications**

1. Facteur de croissance transformant de type $\beta$ purifié ayant la forme d'une protéine homogène, sensible à un séquençage en acides aminés par la dégradation de Edman, et qui peut être obtenu par les étapes de :
   a) extraction de cellules ou tissus murins, humains ou bovins par l'acide-éthanol pour produire un extrait cellulaire ;
   b) dialyse de l'extrait cellulaire pour produire un extrait à l'acide-éthanol dialysé ;
   c) application de l'extrait à l'acide-éthanol dialysé sur une colonne de chromatographie par filtration sur gel et élution avec de l'acide acétique 1M pour obtenir un éluat de filtration sur gel ;
   d) addition de l'éluat de filtration sur gel sur une colonne $C_{18}$ de HPLC en phases inversées utilisant un gradient d'acétonitrile dans de l'acide trifluoroacétique à 0,1 % pour produire un éluat $C_{18}$ ;
   e) application de l'éluat $C_{18}$ sur une colonne de HPLC en phase inversée CN utilisant un gradient de n-propanol dans de l'acide trifluoroacétique à 0,1 % pour obtenir un éluat CN ; et
   f) récupération dans l'éluat CN d'un TGF-$\beta$ séquençable qui migre comme une protéine de poids moléculaire compris entre 12 500 et 13 000 daltons sur un gel de SDS-PAGE réducteur et comme une protéine de poids moléculaire compris entre 25 000 et 26 000 daltons sur un gel de SDS-PAGE non réducteur.

2. Facteur de croissance selon la revendication 1, provenant d'une cellule modifiée par génie génétique.

3. Facteur de croissance selon la revendication 1, provenant de rein de boeuf.

17

**4.** Facteur de croissance transformant selon l'une des revendications 1 à 3, possédant une séquence partielle d'acides aminés déterminée par la dégradation de Edman, constituée des éléments suivants pour chacune de ses deux sous-unités :

$$
\overset{5}{\text{Ala-Leu-Asp-Thr-Asn-Tyr-CMC-}}\overset{10}{\text{Phe-Ser-Ser-Thr-Glu-Lys-}}\overset{15}{\text{Asn-CMC-}} ,
$$

CMC étant de la semi-cystine ou cystéine déterminée en tant que S-carboxyméthylcystéine.

**5.** Procédé de préparation du TGF-$\beta$ séquençable purifié, provenant d'une cellule ou d'un tissu murin, humain ou bovin, comprenant les étapes de la revendication 1.

**6.** Composition destinée à la stimulation de la prolifération cellulaire et de la réparation tissulaire chez l'animal et l'homme, dont le principe actif est constitué essentiellement par un facteur de croissance transformant de type $\beta$ (TGF-$\beta$) selon l'une quelconque des revendications 1 à 4 en des quantités suffisantes pour stimuler la prolifération cellulaire.

**7.** Composition selon la revendication 6, dans laquelle ledit TGF-$\beta$ est présent une quantité comprise entre 1,0 nanogramme et 1 milligramme par ml de composition.

**8.** Composition selon les revendications 6 ou 7, contenant en outre au moins un véhicule pharmaceutiquement acceptable.

**9.** Composition selon la revendication 8, dans laquelle le véhicule est un collagène purifié et la composition est une suspension destinée à l'application topique.

**10.** Composition selon la revendication 8, dans laquelle le véhicule est un sérum physiologique et la composition est un irrigant.

**11.** Composition selon l'une quelconque des revendications 6 à 10, comprenant en outre comme principe actif au moins un agent activant présent en une quantité suffisante pour activer ledit TGF-$\beta$, l'agent activant étant sélectionné dans le groupe constitué par des facteurs de croissance épidermiques (EGF) et des facteurs de croissance transformants de type $\alpha$ (TGF-$\alpha$), lesquels principes actifs sont présents en une quantité suffisante pour stimuler la prolifération cellulaire.

**12.** Composition selon la revendication 11, utilisée pour la stimulation topique de la prolifération de cellules fibroblastiques chez le mammifère, dans laquelle le TGF-$\beta$ et au moins un agent activant sont présents en des quantités approximativement équimolaires.

**13.** Composition selon la revendication 12, dans laquelle l'agent activant est un EGF ou TGF-$\alpha$.

**14.** Composition selon la revendication 11, dans laquelle le TGF-$\beta$ activé est mélangé avec au moins un facteur de croissance secondaire dans un rapport molaire de 1:0,1-10, de préférence en des quantités approximativement équimolaires.

**15.** Composition selon la revendication 11, dans laquelle le facteur de croissance secondaire est au moins un des éléments du groupe constitué par : des facteurs de croissance d'origine plaquettaire, des facteurs de croissance fibroblastiques, des facteurs de croissance angiogéniques, des facteurs de croissance de type insuline, l'insuline, des facteurs de croissance nerveux et des stéroïdes anabolisants.

**16.** Emploi d'une composition selon l'une quelconque des revendications 6 à 15 pour la préparation d'un médicament destiné au traitement de lésions, brûlures, ulcères et autres traumatismes topiques mammaliens par application topique.

**17.** Emploi de la composition selon la revendication 16, dans lequel le médicament est destiné à une réadministration topique périodique ou continue.

**18.** Emploi d'une composition selon l'une quelconque des revendications 6 à 15 pour la préparation d'un médicament utile dans le traitement des traumatismes systémiques mammaliens par administration systémique.

**19.** Emploi d'une composition selon la revendication 18, dans lequel le médicament est destiné à une réadministration systémique périodique ou continue.

**Patentansprüche**

**1.** Gereinigter Transforming-Wachstumsfaktor vom beta-Typ in Form eines homogenen Proteins, der einer Aminosäuresequenzierung durch Edman-Abbau zugänglich ist und durch folgende Stufen erhältlich ist :
  a) Extrahieren von Zellen oder Geweben von Mäusen, Menschen oder Rindern mit Säure-Ethanol, um einen Zellextrakt herzustellen;
  b) Dialysieren des Zellextrakts, um einen dialysierten Säure-Ethanol-Extrakt herzustellen;
  c) Aufbringen des dialysierten Säure-Ethanol-Extrakts auf eine Gelfiltrations-Chromatographiekolonne und Eluieren mit 1-molarer Essigsäure, um ein Gelfiltrationseluat zu erhalten;
  d) Aufgeben des Gelfiltrationseluats auf eine Umkehrphasen HPLC $C_{18}$-Kolonne unter Verwendung eines Acetonitrilgradienten in 0,1 %iger Trifluoressigsäure, um ein $C_{18}$-Eluat herzustellen;
  e) Aufbringen des $C_{18}$-Eluats auf eine CN-Umkehrphasen HPLC-Kolonne unter Verwendung eines n-Propanolgradienten in 0,1 %iger Trifluoressigsäure, um ein CN-Eluat zu erhalten; und
  f) Gewinnung von sequenzierbarem TGF-$\beta$ aus dem CN-Eluat, der als ein Protein mit einem Molekulargewicht von 12 500 bis 13 000 Dalton auf einem reduzierenden SDS-PAGE-Gel läuft und als ein Protein mit einem Molekulargewicht von 25 000 bis 26 000 Dalton auf einem nicht reduzierenden SDS-PAGE Gel läuft.

**2.** Wachstumsfaktor nach Anspruch 1, der aus einer gentechnologisch transformierten Zelle stammt.

**3.** Wachstumsfaktor nach Anspruch 1, der aus einer Rinderniere stammt.

**4.** Transforming-Wachstumsfaktor nach einem der Ansprüche 1 bis 3 mit einer teilweisen Aminosäuresequenz, bestimmt durch Edman-Abbau, bestehend aus der folgenden Sequenz für jede seiner zwei Untereinheiten :

```
                    5                       10                      15
Ala-Leu-Asp-Thr-Asn-Tyr-CMC-Phe-Ser-Ser-Thr-Glu-Lys-Asn-CMC-,
```

worin CMC Halbcystin oder Cystein bedeutet, das als S-Carboxymethylcystein bestimmt wurde.

**5.** Verfahren zur Herstellung eines gereinigten sequenzierbaren TGF-$\beta$, der aus einer Zelle oder dem Gewebe von Mäusen, Menschen oder Rindern herstammt, das die Schritte des Anspruchs 1 umfaßt.

**6.** Zusammensetzung zum Fördern der Zellvermehrung und der Wiederherstellung von Gewebe bei Tieren und Menschen, dessen aktiver Bestandteil im wesentlichen aus einem Transforming-Wachstumsfaktor vom beta-Typ (TGF-$\beta$) nach einem der Ansprüche 1 bis 4 in Mengen, die zum Fördern der Zellvermehrung ausreichen, besteht.

**7.** Zusammensetzung nach Anspruch 6, worin der TGF-$\beta$ in einem Gehalt von 1,0 ng bis 1 mg pro ml der Zusammensetzung vorhanden ist.

**8.** Zusammensetzung nach Ansprüchen 6 oder 7, die zudem mindestens einen pharmazeutisch verträglichen Träger enthält.

**9.** Zusammensetzung nach Anspruch 8, worin der Träger ein gereinigtes Kollagen ist, und die Zusammensetzung eine Suspension zur lokalen Anwendung ist.

**10.** Zusammensetzung nach Anspruch 8, worin der Träger eine physiologische Kochsalzlösung ist und die Zusammensetzung eine Spülung ist.

**11.** Zusammensetzung nach einem der Ansprüche 6 bis 10, die als weiteren aktiven Bestandteil mindestens ein aktivierendes Mittel in einer Menge, die zur Aktivierung von TFG-$\beta$ ausreicht, enthält, wobei das aktivierende Mittel ausgewählt wird unter epidermalen Wachstumsfaktoren (EGF) und Transforming-Wachstumsfaktoren vom alpha-Typ (TGF-$\alpha$), wobei die aktiven Bestandteile in einer Menge vorhanden sind, die ausreicht, um die Zellvermehrung zu fördern.

**12.** Zusammensetzung nach Anspruch 11, die verwendet wird für das lokale Fördern der Fibroblastenzellvermehrung in Säugetieren, wobei TGF-$\beta$ und mindestens ein aktivierendes Mittel in annähernd äquimolaren Mengen vorhanden sind.

**13.** Zusammensetzung nach Anspruch 12, wobei das aktivierende Mittel EGF oder TGF-$\alpha$ ist.

**14.** Zusammensetzung nach Anspruch 11, wobei der aktivierte TGF-$\beta$ mit mindestens einem sekundären Wachstumsfaktor in einem Molverhältnis von 1:0,1-10, vorzugsweise in etwa äquimolaren Mengen, vermischt ist.

**15.** Zusammensetzung nach Anspruch 11, wobei der sekundäre Wachstumsfaktor mindestens einer aus der folgenden Gruppe ist : von Blutplättchen stammende Wachstumsfaktoren, Fibroblastenwachstumsfaktoren, angiogenetischen Wachstumsfaktoren, Wachstumsfaktoren vom Insulin-Typ, Insulin, Nervenwachstumsfaktoren und anabolische Steroide.

**16.** Verwendung einer Zusammensetzung nach einem der Ansprüche 6 bis 15 für die Herstellung eines Arzneimittels zur Behandlung von lokalen Wunden, Verbrennungen, Geschwüren und anderen Traumata von Säugetieren durch lokale Anwendung.

**17.** Verwendung nach Anspruch 16, wobei das Arzneimittel für die periodische oder kontinuierliche lokale Wiederverabreichung bestimmt ist.

**18.** Verwendung einer Zusammensetzung nach einem der Ansprüche 6 bis 15 zur Herstellung eines Arzneimittels, das zur Behandlung von systemischen Traumata bei Säugetieren durch systemische Verabreichung geeignet ist.

**19.** Verwendung nach Anspruch 18, wobei das Arzneimittel zur periodischen oder kontinuierlichen systemischen Wiederverabreichung formuliert ist.